# EUROPEAN PATENT SPECIFICATION

(11) **EP 3 003 401 B1**
(45) Date of publication and mention of the grant of the patent: **15.01.2020**
(21) Application number: 14728571.2
(22) Date of filing: 05.06.2014
(51) Int. Cl.: A61K 51/10

(54) **PHARMACEUTICAL PREPARATION**
PHARMAZEUTISCHE ZUBEREITUNG
PRÉPARATION PHARMACEUTIQUE

(30) Priority: 05.06.2013 GB 201310028
(43) Date of publication of application: 13.04.2016
(73) Proprietor: Bayer AS, 0283 Oslo (NO)
(72) Inventor: FRENVIK, Janne Olsen, N-0884 Olso (NO); RYAN, Olav B, N-0884 Oslo (NO); CUTHBERTSON, Alan, N-0884 Oslo (NO)
(74) Representative: BIP Patents
(86) International application number: PCT/EP2014/061743
(87) International publication number: WO 2014/195423

(56) References cited:
- WO-A1-2004/091668
- WO-A2-2012/131378
- S Purkl ET AL: "Solid-phase extraction using Empore(TM) Radium Rad Disks to separate radium from thorium", Journal of Radioanalytical and Nuclear Chemistry, 1 June 2003 (2003-06-01), pages 473-480, XP055133922, Dordrecht DOI: 10.1023/A:1024547631964 Retrieved from the Internet: URL:http://rd.springer.com/article/10.1023 %2FA%3A1024547631964
- PURKL S ET AL: "A rapid method for alpha-spectrometric analysis of radium isotopes in natural waters using ion-selective membrane technology", APPLIED RADIATION AND ISOTOPES, ELSEVIER, OXFORD, GB, vol. 59, no. 4, 1 October 2003 (2003-10-01), pages 245-254, XP004458717, ISSN: 0969-8043, DOI: 10.1016/S0969-8043(03)00172-6
- IAEA: "Analytical Methodology for the Determination of Radium Isotopes in Environmental Samples AnAnalytical Methodology for the Determination of Radium Isotopes in Environmental Samples", , 1 December 2010 (2010-12-01), XP055133930, Retrieved from the Internet: URL:http://nucleus.iaea.org/rpst/Reference Products/Analytical_Methods/AQ-19.pdf [retrieved on 2014-08-08]

## Description

### Background

The present invention relates to the field of endoradionuclide therapy, and in particular to alpha-endoradionuclide therapy. More specifically the present invention relates to the safety and efficacy of preparations for use in endoradionuclide therapy, to such preparations and to methods for their preparation, treatment and safe storage. WO 2012131378 discloses method of removing radium daughter nuclide from thorium over alginate beds.

The basic principle of endo-radionuclide therapy is the selective destruction of undesirable cell types, e.g. for cancer therapy. Radioactive decay releases significant amounts of energy, carried by high energy particles and/or electromagnetic radiation. The released energy causes cytotoxic damage to cells, resulting in direct or indirect cell death. Obviously, to be effective in treating disease, the radiation must be preferentially targeted to diseased tissue such that this energy and cell damage primarily eliminates undesirable tumour cells, or cells that support tumour growth.

Certain beta-particle emitters have long been regarded as effective in the treatment of cancers. More recently, alpha-emitters have been targeted for use in anti-tumour agents. Alpha-emitters differ in several ways from beta-emitters, for example, they have higher energies and shorter ranges in tissues. The radiation range of typical alpha-emitters in physiological surroundings is generally less than 100 µm, the equivalent of only a few cell diameters. This relatively short range makes alpha-emitters especially well-suited for treatment of tumours including micrometastases, because when they are targeted and controlled effectively, relatively little of the radiated energy will pass beyond the target cells, thus minimising damage to the surrounding healthy tissue. In contrast, a beta-particle has a range of 1 mm or more in water.

The energy of alpha-particle radiation is high compared to that from beta-particles, gamma rays and X-rays, typically being 5-8 MeV, or 5 to 10 times higher than from beta-particle radiation and at least 20 times higher than from gamma radiation. The provision of a very large amount of energy over a very short distance gives alpha-radiation an exceptionally high linear energy transfer (LET) when compared to beta- or gamma-radiation. This explains the exceptional cytotoxicitiy of alpha-emitting radionuclides and also imposes stringent demands on the level of control and study of radionuclide distribution necessary in order to avoid unacceptable side effects due to irradiation of healthy tissue.

Thus, while very potent, it is important to deliver the alpha-emitting radionuclides to the tumour with little or no uptake in non-disease tissues. This may be achieved analogously to what has been shown when delivering the beta-emitting radionuclide yttrium-90 (Y-90) using a monoclonal antibody conjugated with the chelating molecule DTPA as a carrier, i.e. the clinically used radiopharmaceutical Zevalin® (Goldsmith, S.J, Semin. Nucl. Med. 40: 122-35. Radioimmunotherapy of lymphoma: Bexxar and Zevalin.). Thus, a complex of the radionuclide and the carrier-chelator conjugate is administered. Besides full length antibodies of different origins, other types of proteinaceous carriers have been described, including antibody fragments (Adams et al., A single treatment of yttrium-90-labeled CHX-A"-C6.5 diabody inhibits the growth of established human tumor xenografts in immunodeficient mice. Cancer Res. 64: 6200-8, 2004), domain antibodies (Tijink et al., Improved tumor targeting of anti-epidermal growth factor receptor Nanobodies through albumin binding: taking advantage of modular Nanobody technology. Mol. Cancer Ther. 7: 2288-97, 2008), lipochalins (Kim et al., High-affinity recognition of lanthanide(III) chelate complexes by a reprogrammed human lipocalin 2. J. Am. Chem. Soc. 131: 3565-76, 2009), affibody molecules (Tolmachev et al., Radionuclide therapy of HER2-positive microxenografts using a 177Lu-labeled HER2-specific Affibody molecule. Cancer Res. 15:2772-83, 2007) and peptides (Miederer et al., Preclinical evaluation of the alpha-particle generator nuclide 225Ac for somatostatin receptor radiotherapy of neuroendocrine tumors. Clin. Cancer Res. 14:3555-61, 2008).

Decomposition or "decay" of many pharmaceutically relevant alpha emitters results in formation of "daughter" nuclides which may also decay with release of alpha emission. Decay of daughter nuclides may result in formation of a third species of nuclides, which may also be alpha emitter, leading to a continuing chain of radioactive decay, a "decay chain". Therefore, a pharmaceutical preparation of a pharmaceutically relevant alpha emitter will often also contain decay products that are themselves alpha emitters. In such a situation, the preparation will contain a mix of radionuclides, the composition of which depends both on the time after preparation and the half-lives of the different radionuclides in the decay chain.

The very high energy of an alpha-particle, combined with its significant mass, results in significant momentum being imparted to the emitted particle upon nuclear decay. As a result, when the alpha particle is released an equal but opposite momentum is imparted to the remaining daughter nucleus, resulting in "nuclear recoil". This recoil is sufficiently powerful to break most chemical bonds and force the newly formed daughter nuclide out of a chelate complex where the parent nuclide was situated when decomposing. This is highly significant where the daughter nucleus is itself an alpha-radiation emitter or is part of a continuing chain of radioactive decay.

Due to the recoil effects discussed above and due to the change in chemical nature upon radioactive decay, the daughter nuclides thus formed from radioactive decay of the initially incorporated radionuclide may not complex with the chelator. Therefore, in contrast to the parent nuclide, daughter nuclides and subsequent products in the decay chain may not be attached to the carrier. Thus, storage of an alpha-emitting radioactive pharmaceutical preparation will typically lead to accumulation "ingrowth" of free daughter nuclides and subsequent radionuclides in the decay chain, which are no longer effectively bound or chelated. Unbound radioisotopes are not controlled by the targeting mechanisms incorporated into the desired preparation and thus it is desirable to remove the free daughter nuclides prior to dose administration to patients.

Since the radioisotope thorium-227 will be generated and purified in a dedicated production facility, a certain storage period between formation, transportation, complexation and administration of the dose is inevitable, and it is desirable that the pharmaceutical preparation be as free from daughter nuclides as possible as is practicable. A significant problem with past methods has been to administer a reproducible composition of a targeted alpha-radionuclide, which does not contain variable amounts of non-targeted alpha-radionuclides (e.g. free daughter nuclides) in relation to the targeted amount. It is further desirable to reduce the exposure of organic components such as binding/targeting moieties and/or ligands to ionising alpha-radiation. Removal of free radioisotopes from solution contributes to reducing the radiolysis of such components and thus helps to preserve the quality of the pharmaceutical preparation or precursor solution.

Although the decay of the desired nuclide during the storage and transportation period can be calculated and corrected for, this does not avoid the build-up of un-targeted daughter products which can render the composition more toxic and/or reduce the safe storage period and/or alter the therapeutic window in undesirable ways. In addition, it would thus be of benefit for the compositions to be as free from daughter nuclides as possible and that a process for drug product dose manufacture is established which ensures the injected dose has a composition which can be assured as being acceptably safe.

The events following decomposition of thorium-227 may be considered as an illustration of the challenge.

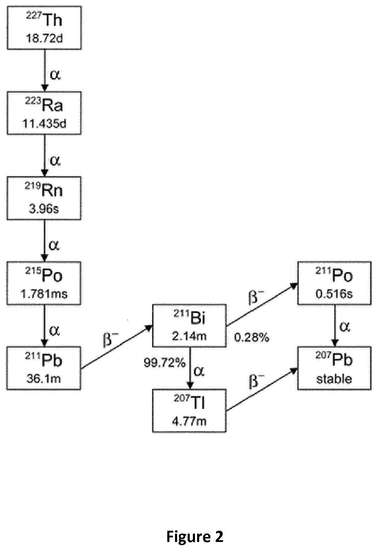

Figure 2 shows the decay chain of thorium-227.

With a half-life of about 18.7 days thorium-227 decomposes into radium-223 upon release of an alpha-particle. Radium-223 in turn has a half-life of about 11.4 days, and decomposing into radon-219, giving rise to polonium-215, which gives rise to lead-211. Each of these steps gives rise to alpha-emission and the half-lives of radon-219 and polonium-215 are less than 4 seconds and less than 2 milliseconds, respectively. The end result is that the radioactivity in a freshly prepared solution of e.g. chelated thorium-227 will increase over the first 19 days, and then start to decrease. Clearly the amount of thorium-227 available for being targeted to a tumor is constantly decreasing, and thus the fraction of the total radioactivity deriving from thorium-227 is dropping during these 19 days, when an equilibrium situation is reached. If daughter nuclides could be specifically removed in a simple procedure, only the amount of thorium-227 (e.g. complexed to the biomolecule carrier) would have to be considered, and the therapeutic window - the relation between therapeutic effect and adverse effects would be unrelated to the time of storage prior to removal of the daughter isotopes. This may be continuous during the storage of the product or may be shortly before administration, such as at the fime of formulation and complexation leading to drug product.

Thus, there is considerable ongoing need for improved radiotherapeutic compositions (particularly for alpha-emitting radionuclides), and procedures for making a solution ready for injection whose biological effects may be reproducibly assessed, without having to consider ingrown radionuclides formed in the radioactive decay chain. Furthermore, there is a need for radiotherapeutic methods and kits allowing facile preparation of a final radioactive formulation under sterile conditions directly prior to administration to a patient. In addition, it is desirable with a view to producing high quality commercial products that meet the rigorous standards of the cGMP principles that the manufacturing process is amenable to automation with minimal manual intervention during dose preparation.

The present invention relates to compositions, methods and procedures for removal of cationic daughter nuclides from a radiopharmaceutical preparation containing an alpha-emitting thorium isotope, which may be in solution or stably chelated to an entity comprising a ligand and a targeting moiety, i.e. the alpha-emitting thorium isotope is complexed or complexable to a ligand which is itself conjugated to a targeting moiety (such as an antibody). In particular, the present inventors have surprisingly established that radium isotopes may be safely and reliably captured onto various selective binders selected from the group consisting of cation exchange resins and ceramic hydroxyapatite, either continuously during storage of the alpha-emitting thorium isotope and/or shortly before administration of the alpha-emitting thorium isotope in the form of a radiopharmaceutical. A typical decay chain for ²²⁷Th is described herein and the isotopes indicated in that chain form preferred daughter isotopes which may be removed and/or captured in the various aspects of the present invention. The final therapeutic formulations obtained from application of the invention are suitable for use in the treatment of both cancer and non-cancerous diseases.

Alternative phrased; the invention provides a composition allowing removal of radium isotopes during storage and/or immediately before administration (e.g. injection) wherein ingrown radium isotopes are removed. This leads to minimal co-administration of radium isotopes and hence minimizing radiation dose and radiation damage to normal and non-target tissues.

Thereby, only the concentration and the half-life of the alpha-emitting thorium isotope and of radium isotopes formed in vivo have to be taken into consideration when calculating the radioactive dose obtained by the patient. Most importantly this leads to a reproducible situation with regard to the relation between efficacy and adverse effects. Thus, the available therapeutic window will not change with storage time of the pharmaceutical preparation.

Phrased differently; by applying the invention the relation between desired anti-tumour effects and adverse effects may be directly related to the measured concentration of the alpha-emitting thorium isotope and becomes independent of the time of storage of the pharmaceutical preparation. In situations where the concentration of the alpha-emitting thorium isotope may be determined by measuring one or more parallel emissions of gamma radiation, sufficiently separate from and gamma emission from the daughter emissions, this may be performed using standard equipment at the radiopharmacy. In fact, if the drug product is pure with respect to the alpha-emitting thorium isotope, the relevant dose of the pharmaceutical preparation will depend only on the time after manufacturing and may be tabulated. In principle there is no need for further measurements at the clinic and the corresponding radiopharmaceutical could be handled in analogy to any other toxic pharmaceutical (although such a procedure would counter current practice, which is based on the fact that radioactivity *can* be easily measured). The enablement of this new and simplified procedure for clinical handling of targeted alpha-emitting radiotherapeutics is important.

In a further embodiment, the invention relates to the provision of a kit for pharmaceutical preparation. Kits are typically supplied to the hospital pharmacy or centralised radiopharmacy and may be prepared for administration shortly (e.g. less than 6 hours) or immediately (e.g. less than one hour) before administration. It would be a considerable advantage if purification of the alpha-emitting thorium isotope could be accomplished at the time of readying of the pharmaceutical preparation for administration. It would be a further advantage if that purification could be carried out without undue burden and without complex handling, since all handling of radioactive materials is desirably minimised.

A kit according to the invention may be in the form of a device, e.g. a cassette laboratory, where tubes or vials containing the various reagents are attached, as well as a syringe to contain the final dosage form of the injectable pharmaceutical preparation. The device performs the operations that would else be performed manually.

It has been established by the present inventors that certain selective binding materials, particularly in the form of or immobilised on a solid or gel, will, to a high extent, retain radium isotopes after decay of the alpha-emitting thorium isotope. The selectivity of these materials allows retention of the radium isotopes but allows the complexed alpha-emitting thorium isotope to pass unhindered through the filter or to be left in solution while the daughters are retained. This provides a considerable advantage in the preparation and delivery of high quality radiopharmaceuticals which can be prepared directly or shortly prior to administration but delivered with a relatively low level of contamination from uncomplexed radium isotopes.

### Summary of the Invention

In a first aspect, the invention therefore provides a pharmaceutical process capable of producing a complexed alpha-emitting thorium isotope (optionally in the form of a biomolecule conjugate). Preferably said process comprises as key component a selective binder selected from the group consisting of cation exchange resins and ceramic hydroxyapatite capable of selectively absorbing, binding, complexing or otherwise removing from solution radium isotopes formed during decay of thorium-227.

A key aspect of the present invention is thus a method for generating a purified solution of an alpha-emitting thorium isotope, said method comprising contacting a solution comprising said alpha-emitting thorium isotope complex and at least one radium isotope with at least one selective binder for said radium isotope and subsequently separating said solution of an alpha-emitting thorium isotope complex from said at least one selective binder, wherein the selective binder is selected from the group consisting of cation exchange resins and ceramic hydroxyapatite.

In all aspects of the present invention, the radium isotopes are generally uncomplexed. This may be the result of the kinetic recoil generated upon alpha-decay and/or as a result of differing complexation properties between the alpha-emitting thorium isotope and the radium isotopes.

The inventors have surprisingly established that appropriate selective binding materials (as described herein, e.g. solid-phase resin materials) are highly effective in absorbing unwanted uncomplexed radium isotopes in the preference to complexed thorium optionally conjugated to targeting moieties (such as biomolecules). Consequently, in a second aspect the present invention provides a method for generating an injectable solution comprising at least one complexed alpha-emitting thorium isotope substantially free from radium isotopes, said method comprising contacting a sample with a suitable selective binder, wherein the selective binder is selected from the group consisting of cation exchange resins and ceramic hydroxyapatite. Preferably this contact will be by means of a simple purification/ filtration step yielding highly radiochemically pure pharmaceutical preparations comprising high levels of alpha-emitting thorium isotope complex (optionally conjugated to a targeting moiety). Typically the separation of the labelled thorium-complex (and optionally conjugate) will be followed immediately by a sterile filtration. This is particularly appropriate as the final step prior to administration.

The present invention thus provides a method for the removal of at least one radium isotope from a solution comprising at least one alpha-emitting thorium isotope complex, said method comprising contacting said solution with at least one selective binder for said at least one radium isotope, said selective binder selected from the group consisting of cation exchange resins and ceramic hydroxyapatite.

In the present invention, the alpha-emitting thorium isotope which is desired for administration (the "parent" radionuclide) will be as described herein and will be "complexed" or "in the form of a complex". These terms take their common meaning in that the alpha-emitting thorium isotope will be in the form of a coordination complex comprising a cation of the alpha-emitting thorium isotope and at least one ligand bound thereto. Suitable ligands, including those described herein, are well known in the art.

Since pharmaceutical preparations may be generated from the solutions of the present invention, the invention provides such pharmaceutical preparations. These will comprise a solution of the alpha-emitting thorium isotope and will be substantially free of radium isotopes as indicated herein. In a pharmaceutical preparation of the invention, the alpha-emitting thorium isotope will be complexed by at least one ligand and the ligand will be conjugated to a targeting (specific binding) moiety as described herein. The solutions of the invention may be provided directly in an administration device (such as a syringe, cartridge or syringe barrel) ready for administration, with the invention allowing for purification of the solution into a pharmaceutical preparation at the time of administration and even by the act of administration (e.g. by administration through a suitable specific binder in the form of a syringe filter). Thus the devices of the invention may be administration devices such as syringes. The invention thus provides in another aspect, an administration device comprising a solution as described herein. Such a device may additionally comprise, for example, a filter, such as a sterile filter. Syringe-filters are appropriate for syringes and similar devices.

The invention thus provides an administration device comprising a solution of at least one complexed alpha-emitting thorium isotope and at least one radium isotope, said device further comprising a filter containing at least one selective binder for said radium isotope, wherein the selective binder is selected from the group consisting of cation exchange resins and ceramic hydroxyapatite. Other devices of the invention which will also comprise a solution of alpha-emitting thorium isotope, a ligand, a targeting moiety and a selective binder selected from the group consisting of cation exchange resins and ceramic hydroxyapatite, will be in the form of (preferably disposable) cartridges, cassettes, rotors, vials, ampoules etc which may be used in the methods of the invention, by manual steps and/or by automated procedures in an automated apparatus.

A further key aspect of the present invention is a kit by which a pharmaceutical preparation may be generated. In a further aspect, the invention therefore provides a kit for the formation of a pharmaceutical preparation of at least one alpha-emitting radioisotope, said kit comprising:
i) a solution of said at least one alpha-emitting thorium isotope and at least one radium isotope;
ii) at least one ligand;
ii) a specific binding moiety;
iii) at least one selective binder for said at least one radium isotope, the selective binder selected from the group consisting of cation exchange resins and ceramic hydroxyapatite.

Wherein said alpha-emitting thorium isotope is complexed or complexable by said ligand which is conjugated or conjugatable to said specific binding moiety.

In one embodiment, the alpha-emitting thorium isotope will be complexed by the ligand but may not be conjugated to the specific binding (targeting) moiety. Alternatively, the ligand may be stably conjugated to the targeting moiety and present in a separate vessel from the thorium isotope. Having the organic molecules of the complex (the ligand and/or the targeting moiety) separate from the thorium isotope reduces the radiation damage (e.g. oxidation) of the organic material due to exposure to alpha-irradiation during storage.

In one embodiment, the kit may be provided as two vials. Such a kit comprises a thorium isotope vial and a second vial containing a buffered solution of biomolecule-conjugate suitably conjugated with a ligand (chelate) which complexes thorium-227. Immediately prior to drug product preparation the thorium-227 vial is mixed with the biomolecule-conjugate solution.

The capture of free (uncomplexed) radionuclides, particularly radium isotopes, from a solution containing at least one complexed alpha-emitting thorium isotope and at least one organic component (such as a complexing agent and/or targeting agent) serves to reduce the exposure of the organic component to ionising radiation from the further decay of the free radium isotopes. Correspondingly, also disclosed is a method for reducing the radiolysis of at least one organic component in a solution comprising at least one alpha-emitting thorium isotope complex, at least one radium isotope and at least one organic component (such as a complexing agent and/or targeting agent), said method comprising contacting said solution with at least one selective binder for said radium isotope, the selective binder selected from the group consisting of cation exchange resins and ceramic hydroxyapatite. This method may be illustrated by a reduction in H₂O₂ concentration in the solution.

In all appropriate aspects of the present invention, the radium isotope will typically be "free" in solution. This indicates that the radium isotope is in the form of a dissolved ion and is not (or not to any significant degree) complexed or bound by ligands in the solution. The radium isotope may obviously be bound to the specific binder but generally this will not be in solution (as described herein). As used herein, the term radium isotope takes its common meaning in the art, in that such isotopes are generated directly or indirectly from the decay of another radioisotope. In the present case, at least one radium isotope present in the solutions referred to herein in any and all aspects of the invention will be a direct (first generation) or indirect (second, third or subsequent generation) decay product of the thorium isotope present in the alpha-emitting thorium isotope complex. It is preferably that at least the first generation decay product of the thorium isotope comprised in the alpha-emitting thorium isotope complex will be present in such solutions and will be bound by the selective binder.

### Detailed Description

As described previously it is dependent on time of storage and transportation how much ingrowth of radium isotopes are present in thorium vial at the time of complexation. The radium isotopes however do not effect the complexation of alpha-emitting thorium isotope (thorium-227) to the biomolecule conjugate as the chelate is chosen such that the thorium isotope has a significantly higher affinity for the chelate compared to the radium isotope. In a second, batch-wise process, the radium isotopes are separated from the now thorium-labelled biomolecule by filtration through a specific binder. This may be in the form of a solid-phase filter cartridge.

This process of separation of the alpha-emitting material from the organic ligand and/or targeting moiety has the added advantage of reducing the rate of radiolysis (e.g. of the biomolecule carrier and/or chelating moiety) of the radiopharmaceutical. Because the radioactive product is manufactured 'closer to bedside' than other strategies currently being employed, the material should have higher radiochemical purity and/or higher purity of the organic material (ligand and/or targeting moiety components). This is beneficial in terms of maintaining shelf-life requirements.

The injectable solutions formed or formable by the methods and uses of the invention are highly suitable for use in therapy, particularly for use in the treatment of hyperplastic or neoplastic disease. Pharmaceutical preparations formed or formable by the various methods of the invention form further aspects of the present invention.

As used herein, the term "pharmaceutical preparation" indicates a preparation of radionuclide with pharmaceutically acceptable carriers, excipients and/or diluents. However, a pharmaceutical preparation may not be in the form which will ultimately be administered. For example, a pharmaceutical preparation may require the addition of at least one further component prior to administration and/or may require final preparation steps such as sterile filtration. A further component can for example be a buffer solution used to render the final solution suitable for injection in vivo. In the context of the present invention, a pharmaceutical preparation may contain significant levels of uncomplexed radionuclides resulting from the radioactive decay chain of the desired radionuclide complex which will preferably be removed to a significant degree by a method according to the present invention before administration. Such a method may involve the batch-wise removal (eg. selective binding, chelation, complexation or absorption) of such uncomplexed daughter radionuclides over a significant part of the storage period of the preparation, or may take place at the final stage, immediately before administration.

In contrast to a pharmaceutical preparation, an "injectable solution" or "final formulation" as used herein indicates a medicament which is ready for administration. Such a formulation will also comprise a preparation of complexed thorium isotopes with pharmaceutically acceptable carriers, excipients and/or diluents but will additionally be sterile, of suitable tonicity and will not contain an unacceptable level of uncomplexed radium isotopes. Such levels are discussed in greater detail herein. Evidently, an injectable solution will not comprise any biopolymer component, although such a biopolymer will preferably have been used in the preparation for that solution as discussed herein.

Injectable solutions formed or formable by any of the methods of the present invention form a further aspect of the invention.

The invention provides a simple method or process for purification and preparation of a sterile final formulation of a radioactive preparation ready for administration, using specific binders in the form of absorbent materials and/or filters to capture radium isotopes yielding rapid separation of radium isotopes during storage and/or immediately prior to administration to a patient. The separation may be followed by the sterile filtration performed as the final formulation is drawn into the syringe, subsequently to be used for administration to the patient or may even take place as part of the act of administration.

Implemented as described, the invention provides a simple kit (as described herein) for purification and final formulation of a radioactive medicament for use in therapy. The kits of the invention may for example include a thorium vessel (such as a vial, syringe or syringe barrel) containing a solution of a radioactive thorium salt (e.g. a ²²⁷Th salt), a vessel (e.g. vial) with a pharmaceutical solution (e.g. a ligand conjugated to a targeting moiety such as an antibody or recepor), a filter containing at least one specific binder for the daughter nuclide(s), optionally a sterile filter and a syringe. The components of the kit may be separate or coupled together into one unit or flow cell forming a closed system therefore reducing the likelihood of introducing unwanted byproducts during the manufacture. Avoiding steps during which radiochemical contamination can be caused is an obvious advantage of kits having components fully or partially sealed together such that material remains within the kit for as many process steps as possible.

The invention provides for the use of the procedure for preparation of a final formulation for injection, for example using components provided as a kit. The procedure of any of the methods and/or uses of the invention may include an incubation step where the solution or pharmaceutical preparation is mixed for example by gentle shaking, to enable optimal complexation of thorium with the biomolecule-chelate conjugate, followed by filtration to remove radium isotopes.

One example procedure for the formation of an injectable solution of an alpha-emitting thorium isotope comprises the steps of:
a) Combining a first solution comprising a dissolved salt of an alpha-emitting thorium isotope and at least one radium isotope with a second solution comprising at least one ligand conjugated to at least one targeting moiety;
b) Incubating the combined solutions at a suitable temperature (e.g. 0°C to 50°C, preferably 20°C to 40°C) for a period to allow complex formation between said ligand and said thorium isotope whereby to form a solution of at least one complexed alpha-emitting thorium isotope;
c) Contacting said solution of at least one complexed alpha-emitting thorium isotope with at least one selective binder for at least one of said radium isotopes, the selective binder selected from the group consisting of cation exchange resins and ceramic hydroxyapatite;
d) separating said solution of at least one complexed alpha-emitting thorium isotope from said at least one selective binder.

In the method of formation of an injectable solution, steps c) and d) constitute a purification method which may be in accordance with any of the appropriate embodiments of the invention as described herein. In this embodiment, the nuclides, binders, ligands and all appropriate aspects will be as indicated herein.

The pharmaceutical preparations of the invention, along with the purified solutions generated by the methods of the invention and the injectable solutions formed by the methods of the invention will desirably have a low concentration of uncomplexed radium isotopes. Typically, for example, the solution concentration of radium isotopes should preferably contribute no more than 10% of the total count of radioactive decays per unit time (from the solution), with the remainder being generated by decay of the complexed thorium isotope. This will preferably be no more than 5% of the total count and more preferably no more than 3%.

Preferably the thorium isotope conjugates of this invention contain thorium-227 wherein the process is most effective in removing ²²³Ra. Other daughter isotopes as indicated herein may also be removed. In the pharmaceutical preparations of the invention and correspondingly in the resulting solutions for injection, as well as in all aspects of the invention, the thorium isotope is complexed or complexable by means of a suitable complexing/chelating entity (generally referred to herein as a ligand). Many suitable ligands are known for the various suitable alpha-emitting radionuclides, such as those based on on DOTA (1,4,7,10-tetraazacyclododecane-1,4,7,10-tetraacetic acid) and other macrocyclic chelators, for example containing the chelating group hydroxy phthalic acid or hydroxy isophthalic acid, as well as different variants of DTPA (diethylene triamine pentaacetic acid), or octadentate hydroxypyridinone-containing chelators. Preferred examples are chelators comprising a hydroxypyridinone moiety, such as a 1,2 hydroxypyridinone moiety and/or a 3,2- hydroxypyridinone moiety. These are very well suited for use in combination with ²²⁷Th. In one embodiment of the invention, the alpha-emitting thorium isotope complex is an octadentate 3,2-HOPO complex of a ²²⁷Th ion.

In the pharmaceutical preparations of the invention and correspondingly in the resulting solutions for injection and all other aspects of the invention, the at least one complexed alpha-emitting thorium isotope is preferably conjugated or conjugateable to at least one targeting moiety (also described herein as a specific binding moiety). Many such moieties are well known in the art and any suitable targeting moiety may be used, individually or in combination. Suitable targeting moieties include poly- and oligo-peptides, proteins, DNA and RNA fragments, aptamers etc. Preferable moieties include peptide and protein binders, e.g. avidin, strepatavidin, a polyclonal or monoclonal antibody (including IgG and IgM type antibodies), or a mixture of proteins or fragments or constructs of protein. Antibodies, antibody constructs, fragments of antibodies (e.g. Fab fragments, single domain antibodies, single-chain variable domain fragment (scFv) etc), constructs containing antibody fragments or a mixture thereof are particularly preferred.

Antibodies, antibody constructs, fragments of antibodies (e.g. Fab fragments or any fragment comprising at least one antigen binding region(s)), constructs of fragments (e.g. single chain antibodies) or a mixture thereof are particularly preferred. Suitable fragments particularly include Fab, F(ab')2, Fab' and/or scFv. Antibody constructs may be of any antibody or fragment indicated herein.

In addition to the various components indicated herein, the pharmaceutical preparations may contain any suitable pharmaceutically compatible components. In the case of radiopharmaceuticals, these will typically include at least one stabiliser. Radical scavengers such as ascorbate, p-ABA and/or citrate are highly suitable. Serum albumin, such as BSA, is also a suitable additive, particularly for protection of protein and/or peptide components such as antibodies and/or their fragments.

In the methods and uses of the present invention, the contacting between the solution part of the pharmaceutical preparation and the selective binder, the selective binder selected from the group consisting of cation exchange resins and ceramic hydroxyapatite may take place over an extended period of time (e.g. at least 30 minutes, such as at least one 1 hour or at least 1 day). In this embodiment, the selective binder may be present with the solution of alpha-emitting thorium isotope during storage. In an alternative embodiment, however, said contacting will occur rapidly (such as over less than 30 minutes, less than 10 minutes, or less than 5 minutes (e.g. less than 1 minute or no more than 30 seconds). In such an embodiment, the selective binder will typically be in the form of or bound to a solid material (as described herein) and may be formed into a separation column, pad or filter through which the solution may be passed. Such passage may be under gravity or by centrifugal force, may be driven by suction or most preferably will be driven by positive pressure, such as by application of pressure to a syringe barrel. In such a case, the contacting takes place.as the solution is pushed through the filter/pad/column. Although rapid separation is the most preferred method, alternatively, the contacting and filtration step may be carried out over longer time periods (e.g. 3 to 20 minutes) to ensure maximum radiochemical purity.

In an alternative embodiment, said contacting/ filtering takes place for no more than 30 seconds preferably no more than 1 minute followed by a sterile filtration and will thus also generate a sterile solution suitable for injection. Correspondingly, the kits of the invention may optionally and preferably additionally comprise a filter (e.g. of pore size 0.45 µm or of pore size of about 0.22 µm). In all cases filtration through a filter of pore size no larger than 0.45 µm, preferably no larger than 0.22 µm is preferred. Such a filter may serve to retain the selective binder employed in the various aspect of the invention.

In the various aspects of the present invention, the ligand moiety is generally conjugated or conjugatable to at least on specific binding (targeting) moiety. Such conjugation may be by means of a covalent bond (such as a carbon-carbon, amide, ester, ether or amine bond) or may be by means of strong non-covalent interactions, such as the binding of a pair of specific-binding moieties, such as biotin to avidin / streptavidin. Most preferably the ligand is conjugated to the targeting moiety by means of a covalent bond, optionally by means of a linker (such as a C1 to C10 alkyl group independently substituted at each end by an alcohol, acid, amine, amide, ester or ether group)

In all aspects of the present invention, the selective binder is typically a solid or gel, or is immobilised on a solid or gel matrix (such as a porous matrix or membrane). This allows for ease of handling and separation and also for ease of contacting the selective binder with the alpha-emitting thorium isotope complex and subsequent separation. A "solid" material may be taken as one which will hold its shape under gentle mechanical pressure including that provided by manual use of a syringe or by the pressure provided in an automated apparatus. Typically the selective binder will be in the form of or immobilised to a porous material such that the solution can pass though the pores of the material. Suitable matrices for supporting selective binders are discussed herein and will be well known to those of skill in the art. These include metal oxides (e.g. silica, alumina, titania) glass, metal, plastics etc. selective binders may be immobilised on the surface of such matrices or may form porous matrices in themselves. Any of the materials indicated may form a support in the form of membranes, resin beads, gel beads, self-assembled lipid structures (e.g. liposomes), microparticles, nanoparticles, powders, crystals and polymer structures as appropriate. Evidently more than one such structure may be used.

As the selective binding material will selected from the group consisting of cation exchange resins, and hydroxyapatite.

Details of inventive and alternate materials suitable for use as selective binding agents are indicated below in Table 1.

**Table 1:**

| Material | Description |
|---|---|
| Cation exchange resins | An insoluble matrix normally in the form of small beads, usually white or yellowish, fabricated from an organic polymer substrate. The material has highly developed structure of pores on the surface of which are sites with easily trapped and released ions. The trapping of ions takes place only with simultaneous releasing of other ions; thus the process is called ion-exchange. |
| Size exclusion/gel filtration resins | Size-exclusion chromatography (SEC) is a chromatographic method in which molecules in solution are separated by their size, and in some cases molecular weight. |
| Molecular sieves | material containing tiny pores of a precise and uniform size that is used as an adsorbent |
| Alginate | (= salts of alginic acid) linear copolymer with homopolymeric blocks of (1-4)-linked β-D-mannuronate (M) and its C-5 epimer α-L-guluronate (G) residues, respectively, covalently linked together in different sequences or blocks |
| Liposomes (sterically stabilized) | artificially-prepared vesicle primarily composed of a lipid bilayer. Liposomes are composed of natural phospholipids, and may also contain mixed lipid chains with surfactant properties. A |
| | liposome design may employ surface ligands. |
| (Poly-) phosphonate | Phosphonates or phosphonic acids are organic compounds containing C-PO(OH)2 or C-PO(OR)2 groups (where R=alkyl, aryl). Phosphonic acids are known as effective chelating agents. The introduction of an amine group into the molecule to obtain -NH2-C-PO(OH)2 increases the metal binding abilities of the phosphonate. |
| Nano-particles | nanoparticles are sized between 100 and 1 nanometers. Large surface to volume ratio. Liposomes are an example of nanoparticles. |
| Phospho-lipids | A class of lipids that are a major component of all cell membranes as they can form lipid bilayers. Most phospholipids contain a diglyceride, a phosphate group, and a simple organic molecule as choline. |
| Glycolipids | lipids with a |
| | carbohydrate attached |
| Co-precipitation | The carrying down by a precipitate of substances normally soluble under the conditions employed. Since the trace element is too dilute (sometimes less than a part per trillion) to precipitate by conventional means, it is typically coprecipitated with a *carrier*, a substance that has a similar crystalline structure that can incorporate the desired element. Occurs by inclusion, adsorption or occlusion. |
| Ferritin/ Apoferritin, transferrin / apotransferrin | Ferritin is a globular protein complex keeping iron in a soluble and non-toxic form. Ferritin that is not combined with iron is called apoferritin. Transferrins are iron-binding blood plasma glycoproteins that control the level of free iron in biological fluids. |
| Lipo-proteins | a biochemical assembly that contains both proteins and lipids |
| Cyclo-dextrines | cyclic oligosaccharides |
| Phytic acid (phytate when in salt form) | Phosphorus compound with chelating actions. It occurs naturally in plants as the insoluble calcium magnesium salt and is a major source of phosphate in the diet, although there is debate about its bioavailability. Excess intake of phytate has been associated with deficiencies of elements such as calcium, iron, and zinc. |
| Surface modifications | Agents with possible affinity for 223-Ra: |
| | Phytic acid |
| | Phospholipids |
| | Phosphonates |
| | Carriers: |
| | Liposomes |
| | Mikroparticles/ nanoparticles/ resins/ alginate/ polymer beads/ |
| | cyclodextrines |

In one aspect, the selective binder(s) are in the form of a column or filter. In this and other appropriate embodiments, the means of contacting will be the flow of solution through or past the selective binder. Alternatively, where the selective binder is immobilised on a support then the flow may be through or past such a support. Subsequent flow through a sterile-filtration membrane (as described herein) is preferred.

The injectable solution obtained from compositions or pharmaceutical formulations of the invention are suitable for treatment of a range of diseases and are particularly suitable for treatment of diseases relating to undesirable cell proliferation, such as hyperplastic and neoplastic diseases. For example, metastatic and non-metastatic cancerous diseases such as small cell and non-small cell lung cancer, malignant melanoma, ovarian cancer, breast cancer, bone cancer, colorectal cancer, pancreatic cancer, bladder cancer, cervical cancer, sarcomas, lymphomas, leukemias, tumours of the prostate, and liver tumours are all suitable targets. The "subject" of the treatment may be human or animal, particularly mammals, more particularly primate, canine, feline or rodent mammals.

Other aspects of the invention are the provision of a composition according to the invention, or alternatively the use of a composition according to the invention in the manufacture of a medicament for use in therapy. Such therapy is particularly for the treatment of diseases including those specified herein above. By "treatment" as used herein, is included reactive and prophylactic treatment, causal and symptomatic treatment and palliation.

Use of the medicament resulting from the invention in therapy may be as part of combination therapy, which comprises administration to a subject in need of such treatment an injectable solution according to the invention and one or more additional treatments. Suitable additional treatments include surgery, chemotherapy and radiotherapy (especially external beam radiotherapy).

In a further aspect the invention encompasses apparatus, kit as described herein. Such kits will comprise an alpha-emitting thorium isotope, a ligand, a targeting moiety and a selective binding material for binding radium isotopes, the selective binder selected from the group consisting of cation exchange resins and ceramic hydroxyapatite. Typically, in use, the alpha-emitting thorium isotope will either be present as an alpha-emitting thorium isotope complex, or will be formed into such complex by contact between a first solution of said kit (comprising the alpha-emitting thorium isotope and any radium isotopes) and a second solution of said kit (comprising the ligand conjugated to the targeting moiety). Following conjugation the alpha-emitting thorium isotope complex will be contacted with the selective binder, the selective binder selected from the group consisting of cation exchange resins and ceramic hydroxyapatite. That contact may be in any way described herein, but will preferably be by passing the alpha-emitting thorium isotope complex solution through a column, pad, filter, membrane or plug of selective binding material.

The kits of the present invention will generally include the selective binding material in the form of a filter or column, the selective binder selected from the group consisting of cation exchange resins and ceramic hydroxyapatite. The alpha-emitting thorium isotope solution will be present in a first vessel but this and all vessels referred to herein may be a vial, syringe, syringe barrel, cartridge, cassette, well, ampoule or any other appropriate vessel as well as a part of such a vessel, such as one well in a plate or one void within a multi-reagent cartridge or cassette. The first and second vessels, where present, may form part of the same device (e.g. may be separate wells or voids in a multi-component plate or cassette) and may be in fluid communication with each other, optionally by means of removing a seal, plug or opening a tap or removing a restriction, clamp etc to allow mixing of solutions. Such mixing may be initiated manually or may be the result of a manipulation within an automated apparatus.

One embodiment of the kits of the invention are in the form of cartridges for an automated apparatus, for example, an automated synthesiser. Such automated apparatus allow for performing the methods of the invention with minimal manual intervention to ensure compliance with cGMP principles. Thus, a typical apparatus includes an automated synthesiser such as the GEHC FastLab or TracerLab which will contain or be loaded with the kit or device of the present invention. An automated apparatus comprising a kit or device of the invention thus forms a further aspect of the invention. The kit of the invention may be in the form of a device, cartridge, rotor, reagent pack etc for any of these or any similar apparatus. An automated apparatus may be used for fully automated process comprising radionuclide (thorium-227) complexation to a ligand/biomolecule conjugate, removal of radium isotopes by filtration on a selective binder, the selective binder selected from the group consisting of cation exchange resins and ceramic hydroxyapatite, sterile filtration and dispensing into a drug product vial. Thus, the various methods of the invention may be carried out by means of an automated apparatus such as one containing a kit or device as described herein.

In a related embodiment, the invention provides for an administration device. Such a device may contain a solution of alpha-emitting thorium isotope complex and radium isotopes and will comprise a selective binder for said radium isotopes, the selective binder selected from the group consisting of cation exchange resins and ceramic hydroxyapatite. In use, such an administration device may concomitantly remove daughter nuclides by passage of the solution through or past the selective binder and also deliver the resulting purified solution to a subject.

The injectable solutions formed and formable from the pharmaceutical compositions of the invention and those formed by use of the kits of the invention will evidently form a further aspect of the invention. Such solutions may be, for example an injectable solution comprising a solution of at least one complexed alpha-emitting thorium isotope and at least one pharmaceutically acceptable carrier or diluent wherein the solution concentration of any uncomplexed radium isotopes resulting from the radioactive decay chain of said least one complexed alpha-emitting thorium isotope is no greater than 10% of the solution concentration of said least one complexed alpha-emitting thorium isotope.

One aspect of the present disclosure relates to a method for reducing the radiolysis of at least one organic component in a solution. Generally this will be a solution as described herein in respect of any embodiment and may comprise at least one alpha-emitting radionuclide complex, at least one daughter radionuclide and at least one organic component. Typically in this and all embodiments, the daughter will be a daughter isotope formed by radioactive decay of at least one alpha-emitting radionuclide in or from a corresponding complex. The organic material may be any organic component including any pharmaceutically acceptable carrier, diluent, buffer etc (any of which, organic or not, may be incorporated into the solutions described in relation to the present invention). Most commonly the organic component will comprises a complexing agent and/or targeting agent, which will typically be the complexing agent of the said complex as described herein. The targeting agent may be any suitable targeting moiety (such as an antibody, antibody fragment (Fab, F(ab')₂ scFv etc), antibody or fragment conjugates etc). The targeting agent will typically be conjugated to the complex by covalent or non-covalent conjugation. By contacting such a solution at least one selective binder for the at least one daughter nuclide (especially at least one selective binder as described in any embodiment herein but most particularly inorganic binders such as hydroxyapatite) then the daughter radionuclides may be sequestered out of solution and separated from both the organic material and other materials, including water, that can readily be ionised or converted into a radical form. As well as direct benefit from reduced direct radiolysis, this reduction in radiolysis will evidently also be an indirect benefit in that the lower concentration of radical and oxidising species will reduce undesirable reactions with the organic material of the complex or targeting moiety. Also disclosed is a method for reducing the H₂O₂ concentration in a solution comprising at least one alpha-emitting radionuclide complex, at least one daughter radionuclide and optionally at least one organic component (such as a complexing agent and/or targeting agent), said method comprising contacting said solution with at least one selective binder for said at least one daughter nuclide.

In all aspects, "reducing" radiolysis or the concentration of a component relates to a reduction in comparison with a control solution containing all corresponding component of the solution except for the specific binder(s). Similarly, "removing" relates to removing a radionuclide from free solution, such as by entrapping that radionuclide within a separable material such as a gel or solid (such as a ceramic, porous solid etc).

The invention will now be illustrated by reference to the following non-limiting Examples, and the Figures below, in which:

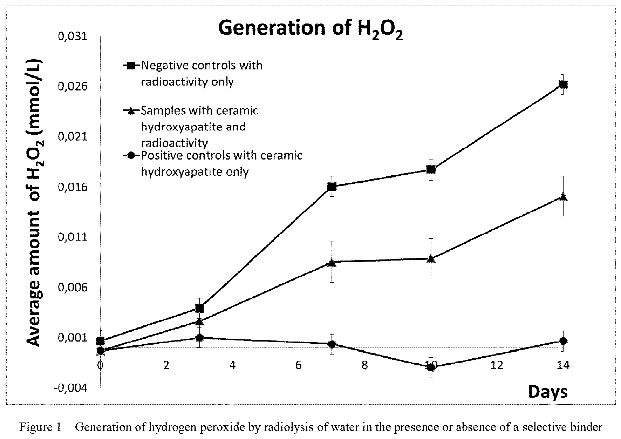

- Figure 1: shows the generation of hydrogen peroxide by radiolysis of water in the presence or absence of a selective binder

### EXAMPLE 1

### Radium-223 uptake on gravity columns using ceramic hydroxyapatite

100 mg ceramic hydroxyapatite was weighed out and transferred to the columns. HEPES buffer (5 mM, pH 8) was used to equilibrate the column (3 x 1 ml). 1 ml HEPES buffer was then added to the column which was left standing over night before 140 kBq radium-223 in 1 mL was loaded. Uptake was immediate. The column was then washed with HEPES buffer (3 x 1 ml), before uptake of radium-223 on the column material was determined using a HPGe-detector instrument (Ortec, Oak Ridge, TN).

The material removed 98.9 % of radium-223 and daughter nuclides (Table 2).

**Table 2 Average percentage retention of radium-223 for ceramic hydroxyapatite (n=3).**

| **Samples** | **Average retention of radium-223 (%)** |
|---|---|
| Ceramic hydroxyapatite | 98.9 |

### EXAMPLE 2

### Purification of a Targeted Thorium Conjugate in phosphate buffer on spin columns with propylsulfonic acid silicabased cation exchange resin

A trastuzumab chelator conjugate prepared as described previously (WO2011/098611A) was labeled with thorium-227 (forming a Targeted Thorium Conjugate, TTC), using thorium-227 stored for 5 days in HCl following purification and hence containing ingrown radium-223 and progenies of radium-223 decay. Each sample contained 0.21 mg TTC, 520 kBq thorium-227 and 160 kBq radium-223 in 300 µl saline phosphate buffer pH 7.4 (Biochrome PBS Dulbecco, Cat no L1825). The sample was added to a column with 15 mg propylsulfonic acid silica based cation exchange resin. The columns were centrifuged (10 000 rcf, 1 min) and the eluate collected. The distribution of thorium-227 (TTC) and radium-223 between the column and eluate was determined using a HPGe-detector instrument (Ortec, Oak Ridge, TN).

The retention of TTC (represented by thorium-227) and radium-223 on the column was 5.5 and 99.1 %, respectively (Table 3).

**Table 3 Retention of Targeted Thorium Conjugate (TTC) and radium-223 after purification on spin columns with cation exchange resin**

| **Amount of cation exchange resin (mg)** | **TTC on column (%)** | **radium-223 on column (%)** |
|---|---|---|
| 15 | 5.5 | 99.1 |

### EXAMPLE 3

### Removal of radium-223 in citrate and phosphate buffer on spin columns with propylsulfonic acid silicabased cation exchange resin

160 kBq radium-223 in 300 µl 50 mM citrate buffer pH 5.5 with 0.9 % sodium chloride or saline phosphate buffer pH 7.4 (Biochrome PBS Dulbecco, Cat no L1825) was added to a column with 60 mg propylsulfonic acid silica based cation exchange resin. The columns were then centrifuged (10 000 rcf, 1 min) and the eluate collected. The distribution of radium-223 between the column and eluate was determined using a HPGe-detector instrument (Ortec, Oak Ridge, TN).

The retention of radium-223 on the column was 96.5 % for the citrate buffer and 99.6 % for the phosphate buffer, respectively (Table 3).

**Table 3 Retention of radium-223 after purification on spin columns with cation exchange resin**

| **Buffer type** | **Average radium-223 on column (%)** |
|---|---|
| Citrate | 96.5 |
| phosphate | 99.6 |

### Example 4 - Further comparison of selective binder materials

Strontium and calcium alginate gel beads, DSPG liposomes, ceramic hydroxyapatite, Zeolite UOP type 4A, and two cation exchange resins (AG50WX8 and SOURCE 30 S) were selected as materials to be studied for radium-223 uptake . Passive diffusional uptake of nuclides was tested by having materials present as suspensions in the formulation. Measurements were taken with the aid of a Germanium detector after 1 hour equilibration at 25 °C with shaking. Removal of free nuclides on gravity columns was also studied.

### Uptake of radium-223

All materials, to some degree, removed radium-223 and daughters by passive diffusional uptake ranging from 30.8 ± 5.8 to 95.4 ± 2.5 % uptake at the selected experimental conditions. All the materials tested removed radium-223 and daughters on the gravity column set-up with near complete uptake. The results were significantly higher (∼ 100 %) and with minimal variation (< 1 %) compared to passive diffusional uptake of radium-223, for all tested materials except for alginate gel beads (see Table 4).

| **Samples** | **Average uptake of radium-223 by passive diffusion (%)** | **Relative standard deviation uptake of radium-223 by passive diffusion(%)** | **Average uptake of radium-223 on gravity column (%)** | **Relative Standard deviation uptake of radium-223 on gravity column (%)** |
|---|---|---|---|---|
| Liposomes | 95.4 | 2.5 | - | - |
| SOURCE 30S cation exchange resins | 78.7 | 15.8 | 99.5 | 0.1 |
| Ceramic hy droxy apatite | 77.8 | 20.1 | A 98.9 | 0.7 |
| Calcium alginate gel beads | 71.9 | 9.7 | 8.2 | 20.7 |
| Strontium alginate gel beads | 68.2 | 16.7 | - | - |
| Zeolite UOP type 4A | 49.7 | 7.4 | - | - |
| Calcium alginate gel beads | 33.1 | 1.7 | - | - |
| AG50WX8 cation exchange resins | 30.8 | 5.8 | 99.8 | 0.2 |

Various materials suitable for capturing radium-223 daughter isotopes have been identified. Strontium and calcium alginate gel beads, DSPG liposomes, ceramic hydroxyapatite, Zeolite UOP type 4A, and two cation exchange resins (AG50WX8 and SOURCE 30 S) were tested and all materials were found to remove radium-223 and daughters.
The cation exchange resins and ceramic hydroxyapatite were however excellent when used on gravity columns.

### Example 5 - Reduction in radiolysis

### Abstract

Formation of hydrogen peroxide (H₂O₂) in the water phase of the formulation was studied as a measure of radiolysis in the presence and absence of ceramic hydroxyapatite, which was one of the materials shown to efficiently bind the radionuclides from solution. Radiolysis and formation of free radicals in the water phase may degrade the radionuclide complex thus minimization of the generation and amount of H₂O₂ present is desirable. After 3 days the concentration of H₂O₂ in samples with ceramic hydroxyapatite was significantly lower than the controls, and the uptake of ²²³Ra and ²²⁷Th from solution was near complete.

### Method

The UVmini-1240 single beam spectrophotometer (190 - 1100 nm) from Shimadzo (Kyoto, Japan) was used and light transmittance recorded at 730 nm for analyzes of the H₂O₂ concentration. Photometric mode was used where the absorbance of a sample is measured at a fixed wavelength (n=3). The cuvettes used were Plastibrand disposable 1.5 ml semi-micro (12.5 x 12.5 x 45 mm) cuvettes made of polystyrene.

A 0.5 mg/ml horseradish peroxidase solution and 2 mg/ml peroxidase substrate (2.2'-Azino-bis(3-ethylbenzothiazoline-6-sulfonic acid) diammonium salt) solution were made by dissolution in metal free water. The peroxidase enzyme converts the peroxidase substrate from colorless to a green color with H₂O₂ as substrate. H₂O₂ standards at 1.765, 0.882, 0.441, 0.221, and 0.110 mmol/L H₂O₂ were made by diluting 30 % (w/w) H₂O₂ in metal free water (n=3). The linearity of the standard curve was R²=0.9995.
Samples consisted of 100 mg/ml ceramic hydroxyapatite in 250 µl 9 mg/ml sodium chloride which was loaded with a freshly prepared ²²⁷Th solution to a concentration of 0.5 kBq/µl (n=3).
Two types of control samples were analyzed; one negative control with only ²²⁷Th and no binding material, and one positive control with binding material but no radioactive source (n=3). The negative controls were analyzed to check the homogeneity of the radionuclides in the sodium chloride solution and the amount of H₂O₂ generated in the absence of binding material, while the positive controls were analyzed to see if a significant level of H₂O₂ was developed without the presence of radioactivity.
For calculation of the percentage uptake of radionuclides in ceramic hydroxyapatite samples and homogeneity of radionuclides in the negative controls, each sample or control was measured on the HPGe-detector before 60 µl supernatant was removed. Samples, controls and standards were further prepared for H₂O₂ analysis by mixing 900 µl 9 mg/ml sodium chloride with 50 µl peroxidase substrate solution, 25 µl horseradish peroxidase solution and 25 µl of the respective supernatant from sample, control or standard. The samples, control or standard were carefully mixed and measured immediately by UV-vis spectrophotometry. For radioactive samples and controls, the remaining sample volume was finally measured on the HPGe-detector. Uptake of radionuclides in ceramic hydroxyapatite or homogeneity of radioactivity in the sodium chloride solution was calculated by the aid of HPGe-spectra.
H₂O₂ concentration in the samples, standards and controls were analyzed by UV-vis spectrophotometry at 730 nm, at time points 0, 3, 7, 10 and 14 days.

### Results

The measured level of H₂O₂ formed during 14 days storage in samples of suspended ceramic hydroxyapatite and freshly prepared ²²⁷Th was significantly lowered compared to negative controls without ceramic hydroxyapatite (Fig. 1). The positive controls containing ceramic hydroxyapatite without radioactivity did not show any H₂O₂ formation outside the statistical error of the method (Fig. 1). The passive diffusional uptake of freshly prepared ²²⁷Th in a suspension of ceramic hydroxyapatite was 81 ± 3 % at 90 minutes reaction time. The consecutive uptake of ²²⁷Th and generated ²²³Ra by ceramic hydroxyapatite was 99 ± 5 % and 102±12 %, respectively, when measured after 14 days incubation.
The measured reduction in H₂O₂ demonstrates a reduced production of radicals and oxidising agents due to radiolysis of the containing solution.

## Claims

1. A method for generating a purified solution of at least one alpha-emitting thorium isotope, said method comprising contacting a solution comprising said at least one alpha-emitting thorium isotope complex and at least one radium isotope with at least one selective binder for said radium isotope and subsequently separating said solution of at least one alpha-emitting thorium isotope complex from said at least one selective binder, wherein the selective binder is selected from the group consisting of cation exchange resins and hydroxyapatite.

2. A method as claimed in claim 1 wherein said alpha-emitting thorium isotope is in the form of a complex with a ligand, wherein said ligand is conjugated to a specific binding moiety such as an antibody.

3. A method as claimed in any preceding claim wherein said selective binder is in the form of, or is attached to, a solid support or gel support.

4. A method as claimed in claim 3 wherein said solid or gel support is in the form of, or attached to, least one selected from membranes, resin heads, gel heads, self-assembled lipid structures (e.g. liposomes), microparticles, nanoparticles, powders, crystals, ceramics and polymer structures.

5. A method as claimed in any preceding claim wherein said solution is contacted with said selective binder by means of flow of said solution through or past said selective binder or through or past a support upon which said selective binder is immobilized.

6. A method as claimed in claim 5 wherein said contacting is by means of a filtration in which said solution flows through or past said selective binder or through or past a support upon which said selective binder is immobilized.

7. A method as claimed in claim 6 wherein said filtration further comprises flowing said solution through a sterile filtration membrane.

8. A method as claimed in any preceding claim wherein said contacting takes place for a period of less than 30 minutes, such as less than 10 minutes, e.g. less than 5 minutes or less than 1 minute (e.g. no more than 30 seconds).

9. A method as claimed in any of claims 1 to 4 wherein said solution is contacted with said selective binder by means of addition of said selective binder and said solution to a vessel (e.g. a sealed or partially sealed vessel).

10. A method as claimed in claim 9 wherein said contacting takes place for 30 minutes or longer, (e.g. 1 hour or longer, such as 1 day or longer)

11. A kit for the formation of a pharmaceutical preparation of at least one alpha-emitting thorium isotope complex, said kit comprising:
i) a solution of said at least one alpha-emitting thorium isotope and at least one radium isotope;
ii) at least one ligand;
iii) a specific binding moiety;
iv) at least one selective binder for said at least one radium isotope.
wherein said alpha-emitting thorium isotope is complexed or complexable by said ligand which is conjugated or conjugatable to said specific binding moiety and the selective binder is selected from the group consisting of cation exchange resins and hydroxyapatite.

12. A kit as claimed in claim 11 wherein said solution of said at least one alpha-emitting thorium isotope and at least one radium isotope is present in a first vessel (e.g. vial, syringe etc) and said ligand conjugated to said specific binding moiety is present in a second vessel.

13. A kit as claimed in claim 11 or claim 12 wherein said selective binder is present in the form of at least one filter, such as a syringe filter, through which said solution of alpha-emitting thorium isotope can he passed after complexation by said ligand and optionally after conjugation to said specific binding moiety.

14. A kit as claimed in claim 11 or claim 12 wherein said selective binder is present in the form of or attached to at least one solid or gel support.

15. A kit as claimed in claim 14 wherein said selective binder is present in said first vessel.

16. A kit as claimed in any of claims 11 to 15 wherein said selective binder is arranged to be separated from said solution by the process of administration of said solution.

17. A kit as claimed in claim 16 wherein said solid or gel support is least one selected from membranes, resin beads, gel beads, self-assembled lipid structures (e.g. liposomes), microparticles, nanoparticles, powders, crystals and polymer structures.

18. A kit as claimed in any of claims 11 to 17 additionally comprising a filter and/or an administration device.

19. A kit as claimed in any of claims 11 to 18 comprising a filter of pore size of no larger than 0.22 µm.

20. An administration device comprising a solution of at least one alpha-emitting thorium isotope complex and at least one radium isotope, said device further comprising a filter containing at least one selective binder for said radium isotope, wherein the selective binder is selected from the group consisting of cation exchange resins and ceramic hydroxyapatite.

21. A device as claimed in claim 20 in the form of a disposable syringe and syringe filter.

22. A kit as claimed in any of claims 11 to 19 comprising an administration device comprising a solution of at least one complexed alpha-emitting radionuclide and at least one daughter nuclide, said kit further comprising a selective binder for said daughter nuclide in the form of a filter.

23. A method for the formation of an injectable solution of a thorium isotope complex comprises the steps of:
a) combining a first solution comprising a dissolved salt of an alpha-emitting thorium isotope and at least one radium isotope with a second solution comprising at least one ligand conjugated to at least one targeting moiety;
b) incubating the combined solutions at a suitable temperature (e.g. 0°C to 50°C, preferably 20°C to 40°C) for a period to allow complex formation between said ligand and said alpha-emitting thorium isotope whereby to form a solution of at least one alpha-emitting thorium isotope complex;
c) contacting said solution of at least one alpha-emitting thorium isotope complex with at least one selective binder for said at least one radium isotope, wherein the selective binder is selected from the group consisting of cation exchange resins and hydroxyapatite;
d) separating said solution of at least one alpha-emitting thorium isotope complex from said at least one selective binder.

24. A method for the formation of an injectable solution as claimed in claim 23 wherein steps c) and d) comprise a method for generating a purified solution as claimed in any of claims 1 to 10.

## Patentansprüche

1. Verfahren zum Erzeugen einer gereinigten Lösung von wenigstens einem alphaemittierenden Thoriumisotop, wobei das Verfahren das Inkontaktbringen einer Lösung, die den wenigstens einen alphaemittierenden Thoriumisotopkomplex und wenigstens ein Radiumisotop umfasst, mit wenigstens einem selektiven Bindemittel für das Radiumisotop und anschließendes Trennen der Lösung des wenigstens einen alphaemittierenden Thoriumisotopkomplexes von dem wenigstens einen selektiven Bindemittel umfasst, wobei das selektive Bindemittel aus der Gruppe ausgewählt ist, die aus Kationenaustauscherharzen und Hydroxyapatit besteht.

2. Verfahren nach Anspruch 1, wobei das alphaemittierende Thoriumisotop in Form eines Komplexes mit einem Liganden vorliegt, wobei der Ligand mit einem spezifischen Bindungsteil, wie einem Antikörper, konjugiert ist.

3. Verfahren nach einem der vorhergehenden Ansprüche, wobei das selektive Bindemittel in der Form eines festen Trägers oder Gelträgers vorliegt, oder daran gebunden ist.

4. Verfahren nach Anspruch 3, wobei der feste Träger oder Gelträger in Form von wenigstens einem ausgewählt aus Membranen, Harzbeads, Gelbeads, selbstassemblierten Lipidstrukturen (z. B. Liposomen), Mikropartikeln, Nanopartikeln, Pulvern, Kristallen, Keramiken und Polymerstrukturen vorliegt, oder daran gebunden ist.

5. Verfahren nach einem der vorhergehenden Ansprüche, wobei die Lösung durch Fließen der Lösung durch das selektive Bindemittel oder daran vorbei oder durch einen Träger, auf dem das selektive Bindemittel immobilisiert ist, oder daran vorbei mit dem selektiven Bindemittel in Kontakt gebracht wird.

6. Verfahren nach Anspruch 5, wobei das Inkontaktbringen mithilfe einer Filtration erfolgt, in der die Lösung durch das selektive Bindemittel oder daran vorbei fließt, oder durch einen Träger, auf dem das selektive Bindemittel immobilisiert ist, oder daran vorbei.

7. Verfahren nach Anspruch 6, wobei die Filtration ferner das Fließenlassen der Lösung durch eine sterile Filtrationsmembran umfasst.

8. Verfahren nach einem der vorhergehenden Ansprüche, wobei das Inkontaktbringen für einen Zeitraum von weniger als 30 Minuten stattfindet, wie weniger als 10 Minuten, z. B. weniger als 5 Minuten oder weniger als 1 Minute (z. B. nicht länger als 30 Sekunden).

9. Verfahren nach einem der Ansprüche 1 bis 4, wobei die Lösung durch Zugabe des selektiven Bindemittels und der Lösung in ein Gefäß (z. B. ein verschlossenes oder teilweise verschlossenes Gefäß) mit dem selektiven Bindemittel in Kontakt gebracht wird.

10. Verfahren nach Anspruch 9, wobei das Inkontaktbringen für 30 Minuten oder länger stattfindet (z. B. 1 Stunde oder länger, wie 1 Tag oder länger).

11. Kit zur Bildung einer pharmazeutischen Zubereitung von wenigstens einem alphaemittierenden Thoriumisotopkomplex, wobei das Kit umfasst:
i) eine Lösung des wenigstens einen alphaemittierenden Thoriumisotops und wenigstens eines Radiumisotops,
ii) wenigstens einen Liganden,
ii)einen spezifischen Bindungsteil,
iii) wenigstens ein selektives Bindemittel für das wenigstens eine Radiumisotop,
wobei das alphaemittierende Thoriumisotop durch den Liganden komplexiert oder komplexierbar ist, der mit dem spezifischen Bindungsteil konjugiert oder konjugierbar ist, und das selektive Bindemittel aus der Gruppe ausgewählt ist, die aus Kationenaustauscherharzen und keramischem Hydroxyapatit besteht.

12. Kit nach Anspruch 11, wobei die Lösung des wenigstens einen alphaemittierenden Thoriumisotops und wenigstens einen Radiumisotops in einem ersten Gefäß (z. B. Fläschchen, Spritze usw.) vorliegt, und der mit dem spezifischen Bindungsteil konjugierte Ligand in einem zweiten Gefäß vorliegt.

13. Kit nach Anspruch 11 oder Anspruch 12, wobei das selektive Bindemittel in Form von wenigstens einem Filter, wie einem Spritzenfilter, vorliegt, durch den die Lösung des alphaemittierenden Thoriumisotops nach Komplexbildung durch den Liganden und wahlweise nach Konjugation mit dem spezifischen Bindungsteil geleitet werden kann.

14. Kit nach Anspruch 11 oder Anspruch 12, wobei das selektive Bindemittel in Form von wenigstens einem festen Träger oder Gelträger vorliegt oder daran gebunden ist.

15. Kit nach Anspruch 14, wobei das selektive Bindemittel in dem ersten Gefäß vorliegt.

16. Kit nach einem der Ansprüche 11 bis 15, wobei das selektive Bindemittel so angeordnet ist, dass es durch den Vorgang der Verabreichung der Lösung von der Lösung getrennt wird.

17. Kit nach Anspruch 16, wobei der feste Träger oder Gelträger wenigstens einer ausgewählt aus Membranen, Harzbeads, Gelbeads, selbstassemblierten Lipidstrukturen (z. B. Liposomen), Mikropartikeln, Nanopartikeln, Pulvern, Kristallen und Polymerstrukturen ist.

18. Kit nach einem der Ansprüche 11 bis 17, das zusätzlich einen Filter und/oder eine Verabreichungsvorrichtung umfasst.

19. Kit nach einem der Ansprüche 11 bis 18, das einen Filter einer Porengröße von nicht mehr als 0,22 µm umfasst.

20. Verabreichungsvorrichtung, umfassend eine Lösung von wenigstens einem alphaemittierenden Thoriumisotopkomplex und wenigstens einem Radiumisotop, wobei die Vorrichtung ferner einen Filter umfasst, der wenigstens ein selektives Bindemittel für das Radiumisotop enthält, wobei das selektive Bindemittel aus der Gruppe ausgewählt ist, die aus Kationenaustauscherharzen und keramischem Hydroxyapatit besteht.

21. Vorrichtung nach Anspruch 20 in Form einer Einwegspritze und eines Einwegspritzenfilters.

22. Kit nach einem der Ansprüche 11 bis 19, umfassend eine Verabreichungsvorrichtung, die eine Lösung von wenigstens einem komplexierten alphaemittierenden Radionuklid und wenigstens einem Tochternuklid umfasst, wobei das Kit ferner ein selektives Bindemittel für das Tochternuklid in Form eines Filters umfasst.

23. Verfahren zur Bildung einer Injektionslösung eines Thoriumisotopkomplexes, das die Schritte:
a) Kombinieren einer ersten Lösung, die ein gelöstes Salz eines alphaemittierenden Thoriumisotops und wenigstens ein Radiumisotop umfasst, mit einer zweiten Lösung, die wenigstens einen Liganden umfasst, der mit wenigstens einem Zielteil konjugiert ist,
b) Inkubieren der kombinierten Lösungen bei einer geeigneten Temperatur (z. B. 0 °C bis 50 °C, bevorzugt 20 °C bis 40 °C) für einen Zeitraum, der Komplexbildung zwischen dem Liganden und dem alphaemittierenden Thoriumisotop erlaubt, wodurch einen Lösung von wenigstens einem alphaemittierenden Thoriumkomplex gebildet wird,
c) Inkontaktbringen der Lösung von wenigstens einem alphaemittierenden Thoriumisotopkomplex mit wenigstens einem selektiven Bindemittel für wenigstens eines von dem Radiumisotop, wobei das selektive Bindemittel aus der Gruppe ausgewählt ist, die aus Kationenaustauscherharzen und keramischem Hydroxyapatit besteht,
d) Trennen der Lösung von wenigstens einem alphaemittierenden Thoriumisotopkomplex von dem wenigstens einen selektiven Bindemittel umfasst.

24. Verfahren zur Bildung einer Injektionslösung nach Anspruch 23, wobei Schritt c) und d) ein Verfahren zum Erzeugen einer gereinigten Lösung nach einem der Ansprüche 1 bis 10 umfassen.

## Revendications

1. Procédé de génération d'une solution purifiée d'au moins un isotope de thorium émetteur alpha, ledit procédé comprenant la mise en contact d'une solution comprenant ledit au moins un complexe d'isotope de thorium émetteur alpha et au moins un isotope de radium avec au moins un agent de liaison sélectif pour ledit isotope de radium, puis la séparation de ladite solution d'au moins un complexe d'isotope de thorium émetteur alpha dudit au moins un agent de liaison sélectif, l'agent de liaison sélectif étant choisi dans le groupe constitué de résines d'échange de cations et de l'hydroxyapatite.

2. Procédé selon la revendication 1, dans lequel ledit isotope de thorium émetteur alpha est sous la forme d'un complexe avec un ligand, ledit ligand étant conjugué à un fragment de liaison spécifique tel qu'un anticorps.

3. Procédé selon l'une quelconque des revendications précédentes, dans lequel ledit agent de liaison sélectif est sous la forme de, ou est lié à, un support solide ou un support de gel.

4. Procédé selon la revendication 3 dans lequel ledit support solide ou de gel est sous la forme de, ou lié à, au moins l'un choisi parmi des membranes, des billes de résine, des billes de gel, des structures lipidiques autoassemblées (par exemple, des liposomes), des microparticules, des nanoparticules, des poudres, des cristaux, des céramiques et des structures de polymère.

5. Procédé selon l'une quelconque des revendications précédentes, dans lequel ladite solution est mise en contact avec ledit agent de liaison sélectif au moyen de l'écoulement de ladite solution à travers ou devant ledit agent de liaison sélectif ou à travers ou devant un support sur lequel ledit agent de liaison sélectif est immobilisé.

6. Procédé selon la revendication 5, dans lequel ladite mise en contact est effectuée au moyen d'une filtration dans laquelle ladite solution s'écoule à travers ou devant ledit agent de liaison sélectif ou à travers ou devant un support sur lequel ledit agent de liaison sélectif est immobilisé.

7. Procédé selon la revendication 6, dans lequel ladite filtration comprend en outre l'écoulement de ladite solution à travers une membrane de filtration stérile.

8. Procédé selon l'une quelconque des revendications précédentes, dans lequel ladite mise en contact est effectuée pendant une durée de moins de 30 minutes, par exemple moins de 10 minutes, par exemple moins de 5 minutes ou moins de 1 minute (par exemple, pas plus de 30 secondes).

9. Procédé selon l'une quelconque des revendications 1 à 4, dans lequel ladite solution est mise en contact avec ledit agent de liaison sélectif au moyen de l'ajout dudit agent de liaison sélectif et de ladite solution dans un récipient (par exemple, un récipient scellé ou partiellement scellé).

10. Procédé selon la revendication 9, dans lequel ladite mise en contact est effectuée pendant 30 minutes ou plus (par exemple, 1 heure ou plus, tel que 1 jour ou plus).

11. Trousse pour la formation d'une préparation pharmaceutique d'au moins un complexe d'isotope de thorium émetteur alpha, ladite trousse comprenant :
i) une solution dudit au moins un isotope de thorium émetteur alpha et d'au moins un isotope de radium ;
ii) au moins un ligand ;
ii) un fragment de liaison spécifique ;
iii) au moins un agent de liaison sélectif pour ledit au moins un isotope de radium,
dans laquelle ledit isotope de thorium émetteur alpha est complexé ou peut être complexé par ledit ligand qui est conjugué ou peut être conjugué audit fragment de liaison spécifique et l'agent de liaison sélectif est choisi dans le groupe constitué de résines d'échange de cations et d'hydroxyapatite céramique.

12. Trousse selon la revendication 11, dans laquelle ladite solution desdits au moins un isotope de thorium émetteur alpha et au moins un isotope de radium est présente dans un premier récipient (par exemple, un flacon, une seringue, etc.) et ledit ligand conjugué audit fragment de liaison spécifique est présent dans un deuxième récipient.

13. Trousse selon la revendication 11 ou la revendication 12, dans laquelle ledit agent de liaison sélectif est présent sous la forme d'au moins un filtre, tel qu'un filtre à seringue, à travers lequel ladite solution d'isotope de thorium émetteur alpha peut être passée après complexation par ledit ligand et facultativement, après conjugaison audit fragment de liaison spécifique.

14. Trousse selon la revendication 11 ou la revendication 12, dans laquelle ledit agent de liaison sélectif est présent sous la forme de ou lié à au moins un support solide ou de gel.

15. Trousse selon la revendication 14, dans laquelle ledit agent de liaison sélectif est présent dans ledit premier récipient.

16. Trousse selon l'une quelconque des revendications 11 à 15, dans laquelle ledit agent de liaison sélectif est agencé de façon à être séparé de ladite solution par le processus d'administration de ladite solution.

17. Trousse selon la revendication 16, dans laquelle ledit support solide ou de gel est au moins l'un choisi parmi des membranes, des billes de résine, des billes de gel, des structures lipidiques autoassemblées (par exemple, des liposomes), des microparticules, des nanoparticules, des poudres, des cristaux, des céramiques et des structures de polymère.

18. Trousse selon l'une quelconque des revendications 11 à 17, comprenant en outre un filtre et/ou un dispositif d'administration.

19. Trousse selon l'une quelconque des revendications 11 à 18, comprenant un filtre ayant une taille de pore de pas plus de 0,22 µm.

20. Dispositif d'administration comprenant une solution d'au moins un complexe d'isotope de thorium émetteur alpha et d'au moins un isotope de radium, ledit dispositif comprenant en outre un filtre contenant au moins un agent de liaison sélectif for ledit isotope de radium, dans lequel l'agent de liaison sélectif est choisi dans le groupe constitué de résines d'échange de cations et d'hydroxyapatite céramique.

21. Dispositif selon la revendication 20, sous la forme d'une seringue jetable et d'un filtre à seringue.

22. Trousse selon l'une quelconque des revendications 11 à 19, comprenant un dispositif d'administration comprenant une solution d'au moins un radionucléide émetteur alpha complexé et au moins un nucléide fils, ladite trousse comprenant en outre un agent de liaison sélectif pour ledit nucléide fils sous la forme d'un filtre.

23. Procédé de formation d'une solution injectable d'un complexe d'isotope de thorium qui comprend les étapes de :
a) combinaison d'une première solution comprenant un sel dissous d'un isotope de thorium émetteur alpha et au moins un isotope de radium avec une deuxième solution comprenant au moins un ligand conjugué à au moins un fragment de ciblage ;
b) incubation des solutions combinées à une température adaptée (par exemple 0 °C à 50 °C, de préférence 20 °C à 40 °C) pendant une durée suffisante pour permettre la formation d'un complexe entre ledit ligand et ledit isotope de thorium émetteur alpha de façon à former une solution d'au moins un complexe d'isotope de thorium émetteur alpha ;
c) mise en contact de ladite solution d'au moins un complexe d'isotope de thorium émetteur alpha avec au moins un agent de liaison sélectif pour au moins l'un dudit isotope de radium, dans lequel l'agent de liaison sélectif est choisi dans le groupe constitué de résines d'échange de cations et d'hydroxyapatite céramique ;
d) séparation de ladite solution d'au moins un complexe d'isotope de thorium émetteur alpha dudit au moins un agent de liaison sélectif.

24. Procédé de formation d'une solution injectable selon la revendication 23, dans lequel les étapes c) et d) comprennent un procédé de génération d'une solution purifiée selon l'une quelconque des revendications 1 à 10.
